# EUROPEAN PATENT APPLICATION

(11) **EP 4 450 055 A1**
(43) Date of publication of application: **23.10.2024**
(21) Application number: 22907610.4
(22) Date of filing: 22.08.2022
(51) Int. Cl.: A61K 8/60, A61K 8/9789, A61Q 11/00, A61Q 17/00, A23L 33/125

(54) **COMPOSITION FOR REDUCING DENTAL PLAQUE AND INHIBITING DENTAL PLAQUE ACIDIFICATION, CONTAINING GINSENOSIDE COMPOUND K OR COMPLEX GINSENOSIDE COMPOSITION**

(30) Priority: 15.12.2021 KR 20210179760
(71) Applicant: BTGin Co., Ltd., Daejeon 34024 (KR)
(72) Inventor: OH, Jin-Hwan, Sejong-si, 30100 (KR); HER, Youl, Seoul 06331 (KR); KIM, Jin-Hee, Daejeon 34006 (KR); LEE, Hyun-Cheol, Sejong 30150 (KR); HA, Yoo-Jin, Daejeon 34008 (KR)
(74) Representative: Comoglio, Elena
(86) International application number: PCT/KR2022/012482
(87) International publication number: WO 2023/113139

(57) **Abstract**

[Problem] To provide a natural substance-derived composition which has few or no side effects and exhibits activity that reduces dental plaque and inhibits dental plaque acidification. [Solution] The present inventors found that Ginsenoside compound K derived from red ginseng etc. or a complex ginsenoside composition, in which Ginsenoside compound K, Ginsenoside Rg3 (R+S), Ginsenoside F2, Ginsenoside Rh2, and Ginsenoside Rb1 are mixed, has the remarkable effects of reducing dental plaque and inhibiting the production of organic acid on tooth surfaces. This composition can be used as an oral composition such as toothpaste or mouthwash, and can also be applied to food compositions such as gum, beverages, and confectionery.

## Description

### Technical Field

The present invention relates to a composition, for reducing dental plaque and inhibiting dental plaque acidification, containing a complex ginsenoside composition or ginsenoside compound K, which is extracted and/or processed from ginseng.

The present invention was made with the support of the Ministry of SEMs and startup of the Republic of Korea (Project ID number: 1425147780, Project title: Commercialization and efficacy verification of red ginseng specific saponin extract having anti-cariogenic efficacy).

### Background Art

Caries is one of the most prevalent oral diseases worldwide, and the morbidity rate thereof has been recently increasing due to changes in dietary patterns, such as increased sugar consumption. Dental caries (tooth decay), which is a multifactorial disease caused by the interaction of bacteria, food, and saliva in the plaque, is a bacterial dental hard tissue disease in which the main causative bacteria *Streptococcus mutans (S. mutans)* and *Streptococcus sobrinus (S. sobrinus)* among the bacteria within the dental plaque are attach to the tooth surface to result in tooth demineralization and tooth enamel destruction through proliferation and acid production, thereby causing loss of tooth tissue. For organic acids, produced by the caries-causative bacteria, to act directly on the dentin, acid-producing bacteria essentially remain on the tooth surface, necessitating the formation of insoluble glucans. The glucosyltransferase (GTase) secreted by these bacteria use sucrose to form, as glucose polymers, insoluble glucans composed of 90% or more of α-1,3 glucosidic linkages and the remainder α-1,6 glucosidic linkages. The insoluble glucans have the ability to adhere to the tooth surface without dissolving in water, and thus form plaque to result in bacterial colonization. This bacterial colonization covers the tooth surface to form the dental plaque, and organic acids, such as lactic acid, are accumulated within the dental plaque by caries-causative bacteria, thereby resulting in a lower pH, causing tooth demineralization. The dental plaque, on which calcium and phosphate in saliva are attached, hardens to form calculus, and dental caries is accelerated in proportion to the amount of calculus (Functional Food Evaluation Guidelines, Ministry of Food and Drug Safety, December 2017, Park et al. Kor. J. Food & Nutr. 13(2), 139(2000), and Kim et al. J. Kor. Fish. Soc. 35(2), 191(2002)).

Various methods have been researched for the development of therapeutic and preventive preparations for dental caries, and one of the promising approaches is inhibiting the formation of dental biofilms formed on the teeth (Healthcare Technology Research and Development Report, Biofilm control research for dental caries prevention and treatment, 2013). Additionally, the development of GTase inhibitors to prevent the formation of biofilms (Ren. Z. et al. Antimicrob Agents Chemother. 2015) is one of the biofilm inhibition methods that are frequently used. Furthermore, antimicrobial agents to inhibit the proliferation of caries-causative bacteria are being actively developed for the treatment and prevention of dental caries (J. Periodont. Res., 21(16 suppl.)33(1986), Arch. Oral Biol., 17, 147(1972), J. Periodont. Res., 21(16 suppl.)74(1986)). Especially, the development of antimicrobial active agents using natural extracts with low toxicity and excellent stability is actively progressing, and thus caries inhibitors using an aloe extract (Kor. J. Food & Nutr. 13(2), 139(2000)), a seaweed extract (J. Korean Fish. Soc. 35(2), 191(2002)), a *Schisandra chinensis* extract are researched and developed (Korean Patent No. 10-0296775). These natural extracts use edible plants as raw materials and thus are almost non-toxic and easily consumable. However, natural extracts are in the form of a simple extract of various substances mixed, and as a result, the natural extracts contain trace amounts of specific pharmacologically active ingredients, and the mass-production of the ingredients and the control of the contents of these ingredients are difficult, resulting in poor reproducibility of pharmacological efficacy. Consequently, the development of natural extracts into functional foods or medicinal products is challenging.

Hence, the present inventors have explored an effective naturally derived composition for preventing caries by inhibiting dental plaque formation and dental plaque acidification, which are key mechanisms of dental caries, and a caries prevention method using the same.

### Detailed Description of the Invention

### Technical Problem

An aspect of the present invention is to provide a natural substance-derived composition exhibiting effects of reducing dental plaque and/or inhibiting dental plaque acidification while having few or no side effects.

### Technical Solution

The present inventors revealed that ginsenoside compound K, which is extracted from red ginseng or ginseng, or a complex ginsenoside composition, which is obtained by extracting a complex ginsenoside from red ginseng or ginseng using a solvent, partially purifying the extract by means of adsorption through an adsorption resin followed by desorption with a solvent, and then mixing the resultant substance with a ginseng concentration, had functions of reducing dental plaque and inhibiting dental plaque acidification, and invented a toothpaste composition, a mouthwash composition, a food composition, and a pharmaceutical composition, each containing the above-mentioned compositions.

### Advantageous Effects

The ginsenoside compound K or complex ginsenoside composition of the present invention exhibited effects of reducing dental plaque and/or inhibiting organic acid production on the tooth surface.

Therefore, the ginsenoside compound K or complex ginsenoside composition of the present invention can be used as oral compositions including toothpastes and mouthwashes, and can also be applied to food compositions, such as gum, beverages, and snacks.

### Brief Description of the Drawings

FIG. 1 shows dental plaque reduction experiment results and images of *S. mutans* attached to glass tubes (**p*<0.05, ***p*<0.01, ****p*<0.001, *****p*<0.0001, compared to control).

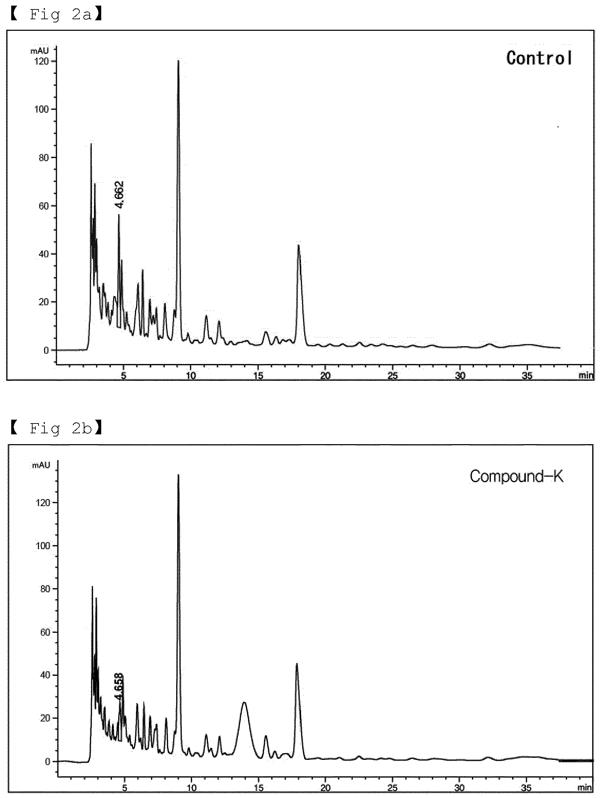

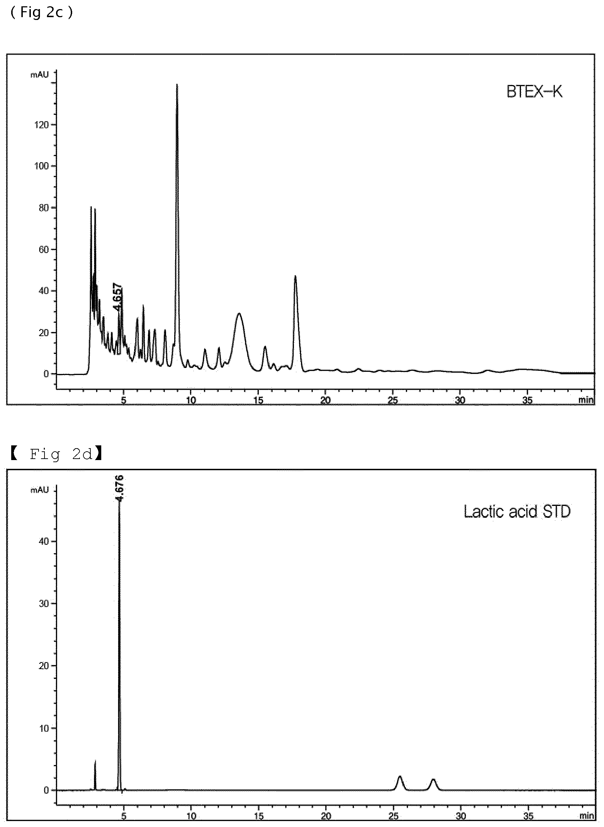

FIG. 2 shows HPLC analysis results of organic acid production.

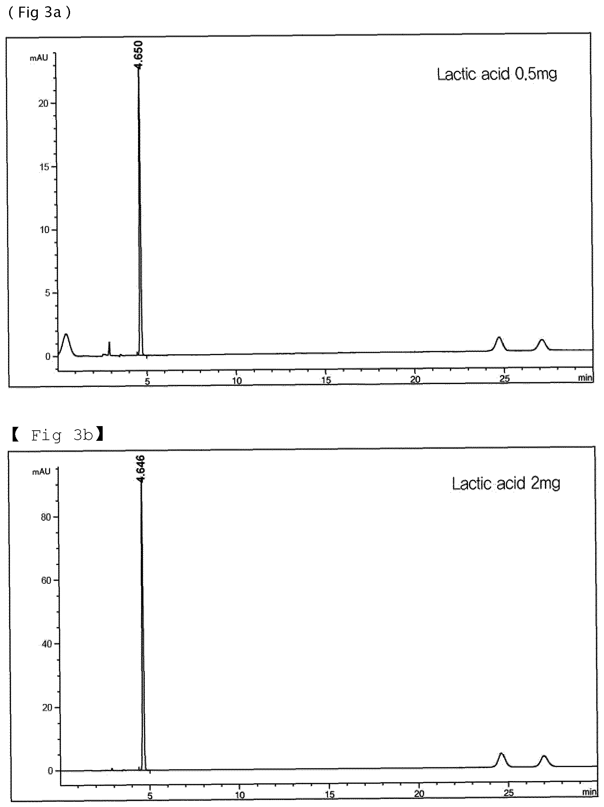

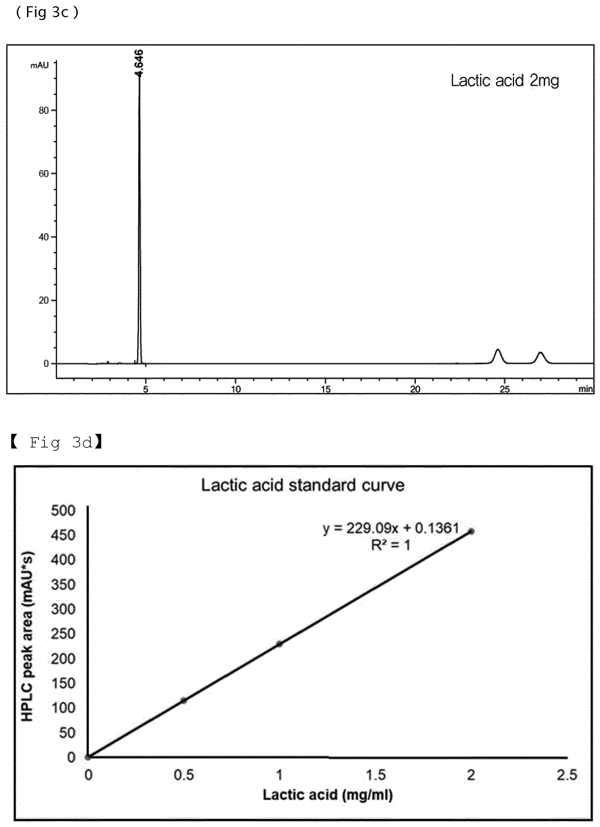

FIG. 3 shows HPLC analysis results of lactic acid standard samples and a lactic acid standard curve with concentration.

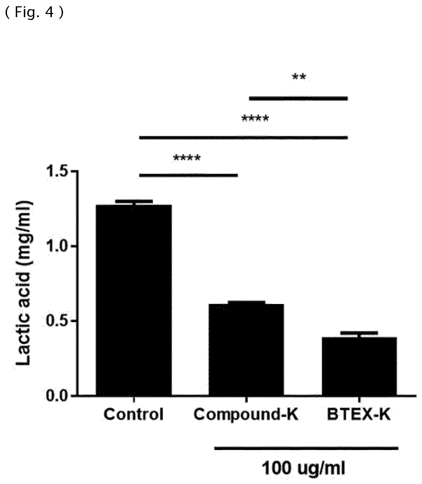

FIG. 4 shows quantitative statistical results of HPLC analysis of organic acid production (****p<0.0001 compared to control; and **p<0.01 compared to compound K and BTEX-K).

### Best Mode for Carrying Out the Invention

The present invention is directed to a composition for reducing dental plaque and inhibiting dental plaque acidification, the composition containing, as an active ingredient,
(A) ginsenoside compound K; or
(B) a complex ginsenoside composition containing, relative to 100 parts by weight of the ginsenoside compound K, ginsenoside Rg3 (R+S), ginsenoside F2, ginsenoside Rh2, and ginsenoside Rb1 at least 7 parts or more by weight each.

Additionally, the complex ginsenoside composition B) may be obtained by steps:
1) subjecting red ginseng to extraction and concentration to obtain a red ginseng concentrate;
2) dissolving the red ginseng concentrate, followed by adsorption through a synthetic adsorption resin column and then desorption with ethanol, thereby obtaining a complex ginsenoside containing ginsenosides Rb1, Rb2, Rc, and Rd;
3) treating the complex ginsenoside with alpha-galactosidase to obtain a fermented complex ginsenoside; and
4) adding the fermented complex ginsenoside obtained in step 3) to the red ginseng concentrate obtained in step 1) to obtain a complex ginsenoside composition.

The synthetic adsorption resin column is not particularly limited, but is preferably a porous adsorption resin column, more preferably, at least one of HP2MG or HP-20.

The complex ginsenoside composition of the present invention may contain ginsenoside compound K at least 100 mg/g or more, and ginsenoside Rg3 (R+S), ginsenoside F2, ginsenoside Rh2, and ginsenoside Rb1 at least 8 mg/g or more each, preferably 8 mg/g to 30 mg/g each, and more preferably 10 mg/g to 30 mg/g or less each.

The red ginseng concentrates in steps 1), 2), and 4) may be in a powder form or a liquid phase.

The ginsenoside compound K or complex ginsenoside composition may be in a liquid phase, a cream phase, a paste phase, or a solid phase.

In accordance with still another aspect of the present invention, there is provided a pharmaceutical composition, for prevention or treatment of caries, containing the composition for reducing dental plaque and inhibiting dental plaque acidification.

The pharmaceutical composition, for prevention or treatment of dental caries, containing as an active ingredient ginsenoside compound K and/or complex ginsenoside composition BTEX-K, may be formulated, in blending with a carrier, which is commonly acceptable in a pharmaceutical field, into a form of an injection, an oral administration, an agent for application, or the like by a common method. For example, the pharmaceutical composition may be formulated into a liquid, a syrup, a capsule, granules, a powder, an ointment, an emulsion, a gel, a cream, or the like, and may be administered through various routes. Among various formulations, for example, a composition for injection is preferably an isotonic aqueous solution or suspension, and the above-mentioned composition is sterile and/or contains an adjuvant (e.g. a preservative, a stabilizer, a wetting or emulsifying agent, a solution promoter, a salt and/or buffer for regulating the osmotic pressure). Additionally, these may contain other therapeutically useful substances. Examples of the pharmaceutically acceptable carrier include lactose, glucose, sucrose, sorbitol, mannitol, starch, gum acacia, alginate, gelatin, calcium phosphate, calcium silicate, cellulose, methyl cellulose, microcrystalline cellulose, polyvinylpyrrolidone, water, methylhydroxybenzoate, propyl hydroxybenzoate, talc, magnesium stearate, and mineral oil. The composition may further contain a lubricant, a wetting agent, a flavoring agent, an emulsifier, a preservative, and the like. The composition of the present invention may be formulated into an oral preparation, such as granules, a powder, a solution, a tablet, a capsule, or a dry syrup, or a parenteral dosage form such as an injection, but is not limited to these dosage forms.

The pharmaceutical preparation thus obtained may be administered through subcutaneous administration, intramuscular administration, oral administration, intravenous administration, oral application, gingival application, or the like according to the purpose. The daily dose of 0.001 µg/kg to 100 mg/kg may be administered once or divided into multiple doses. The dose level for a particular patient may vary depending on the patient's body weight, age, sex, or health condition, the time of administration, the method of administration, the severity of disease, and the like.

Furthermore, the pharmaceutical composition, for prevention or treatment of caries, containing as an active ingredient ginsenoside compound K and/or complex ginsenoside composition BTEX-K, may be, especially, an externally applied agent for gingival application or oral application. An agent for application containing as an active ingredient the composition for prevention or treatment of caries of the present invention can be easily prepared in any form according to a conventional preparation method. As an example, when a cream-type agent for application is prepared, the anti-inflammatory composition of the present invention is allowed to be contained in a general oil-in-water (O/W) or water-in-oil (W/O) cream base, and a flavoring agent, a chelating agent, a pigment, an antioxidant, a preservative, and the like are used as needed, and synthetic or natural substances, such as proteins, minerals, and vitamins, can be used together for the purpose of enhancing properties.

In accordance with still another aspect of the present invention, there is provided a toothpaste composition containing the composition for reducing dental plaque and inhibiting dental plaque acidification.

In accordance with still another aspect of the present invention, there is provided a mouthwash composition containing the composition for reducing dental plaque and inhibiting dental plaque acidification.

In accordance with still another aspect of the present invention, there is provided a food composition containing the composition for reducing dental plaque and inhibiting dental plaque acidification.

The term "food" as used herein refers to a natural or processed product containing one or more nutrients and, preferably, refers a product in a state that is directly edible after a certain level of processing, and in the accepted meaning, the food may include food, a food additive, a functional food, a health functional food, a health supplement food, a beverage, and the like. The food composition of the present invention may be prepared in the form of various kinds of beverages, gums, teas, snacks, vitamin complexes, health supplements, or the like, without limitation thereto. The preferred amount of intake of the food composition of the present invention varies depending on the condition, body weight, or severity of symptoms of a person taking the food composition, the form of food, or the period of intake, and can be selected appropriately. In the food composition of the present invention, it is preferable for optimal effects that the active ingredient is consumed at a dose of 0.2 mg/kg to 200 mg/kg per day.

The present invention is directed to a toothpaste composition containing as an active ingredient the ginsenoside compound K or complex ginsenoside composition. The tooth composition may be formulated by a conventional toothpaste composition preparation method through the addition of a mastic oil, a propolis extract, sodium pyrophosphate, sodium fluoride, tocopherol acetate, hydroxyapatite, a sweetener, a thickener, an abrasive, a wetting agent, a surfactant, or the like, as necessary.

Furthermore, the present invention is directed to a mouthwash composition containing as an active ingredient the ginsenoside compound K or complex ginsenoside composition. The mouthwash composition may be formulated by a conventional mouthwash composition preparation method through the addition of a solubilizer, a wetting agent, a surfactant, a lubricant, a flavoring agent, a sweetener, a preservative, or a medicinal agent, necessary, and can be formulated into a conventional mouthwash preparation method. Also, the mouthwash composition may be prepared into a formulation selected from the group consisting of a gum, a mouthwash, a mouthwash freshener, an oral spray, an oral ointment, an oral patch, or a combination thereof.

### Mode for Carrying Out the Invention

Hereinafter, specific methods of the present invention will be described in more detail with reference to examples. However, it would be obvious to a person skilled in the art to which the present invention pertains that the scope of the present invention is not limited to these examples.

### Example 1: Preparation of complex ginsenoside

Korean dried red ginseng (4 years old) was purchased and used as raw material for experiments (Deokwon Ginseng, Geumsan, Chungcheongnam-do). A complex ginsenoside was extracted from dried red ginseng. A red ginseng concentrate was obtained by subjecting 10 kg of dried red ginseng to extraction and concentration with 50% ethanol at 60°C for 12 hours, repeated three times.

Out of a total of 5.6 kg (63 brix) of the red ginseng concentrate, 5.5 kg was dissolved in water, adsorbed by passing through a column filled with synthetic adsorption resin (HP2MG, Misbish Chemical, or HP-20, Diaion), and then desorbed with 95% ethanol, thereby obtaining 460 g of a complex ginsenoside containing ginsenosides Rb1, Rb2, Rc, and Rd.

### Example 2: Preparation of red ginseng concentrate powder

Out of 5.6 kg of the red ginseng concentrate prepared in Example 1, 100 g was separately taken and concentrated under reduced pressure, thereby obtaining 71 g of a red ginseng concentrate powder.

### Example 3: Preparation of fermented complex ginsenoside

The complex ginsenoside obtained in Example 1 (100 g) as a substrate was reacted with alpha-galactosidase (0.75 g) derived from the *Aspergillus genus* in a 50 mM sodium acetate buffer solution (pH 4.5) in a 60°C constant-temperature water bath for 84 hours, thereby preparing a fermented complex ginsenoside (41 g).

The fermented complex ginsenoside thus prepared contained ginsenoside compound K, ginsenoside F2, ginsenoside Rh2, and the like.

### Example 4: Preparation of complex ginsenoside composition (hereinafter used interchangeably with "BTEX-K")

A complex ginsenoside composition BTEX-K was prepared by mixing 100 g of the fermented complex ginsenoside prepared in Example 3 with 40 g of the red ginseng concentrate powder prepared in Example 2.

The complex ginsenoside composition BTEX-K contained various ginsenosides, such as ginsenoside F2 and ginsenoside Rh2, in addition to ginsenoside compound K (100 mg/g).

**TABLE 1**

| Contents of main ginsenosides in BTEX-K (10 mg/g or more) | | | | | |
|---|---|---|---|---|---|
| Ginsenoside | Rb 1 | F2 | Rg3 (R+S) | Rh2 | Ginsenoside compound K |
| Content (mg/g) | 11.5 | 11.5 | 25.5 | 15.4 | 100 |

### Experimental Example 1: Analysis of dental plaque reduction

1) A brain heart infusion (BHI) broth (5 ml) containing 1% sucrose was added in 50 ml glass tube, and 50 µl of each of sample extracts by concentrations shown in Table 2 below was added. For a control group, sterilized water was used instead of sample extracts.

**TABLE 2**

| NO | Sample | Concentration |
|---|---|---|
| 1 | Ginsenoside compound K | 0.1, 1, 2 mg/ml |
| 2 | Complex ginsenoside composition (BTEX-K) | 0.5, 5, 10 mg/ml |

*2) S. mutans* was inoculated to reach 10⁵.
3) The glass tube was cultured statically in an incubator at 37°C for 24 hours while tilted at 30°.
4) After culturing, 5 ml of 0.5 M NaOH solution was added to suspend the cells attached to the wall.
5) One milliliter of suspension was taken and measured for absorbance at 550 nm.

### Experimental Example 2: Amount of organic acid produced in dental plaque

1) To a brain heart infusion (BHI) broth (2 ml), 20 µl of each of sample extracts by concentrations in Table 3 below was added. For a control group, sterilized water was used instead of sample extracts.
*2) S. mutans* was inoculated to reach 10⁵-10⁶.
3) The cells were cultured in an incubator at 37°C for 48 hours.
4) The amount of the acid secreted in the medium was measured using HPLC.

**TABLE 3**

| NO | Sample | Concentration |
|---|---|---|
| 1 | Ginsenoside compound K | 0.1, 1, 2 mg/ml |
| 2 | Complex ginsenoside composition (BTEX-K) | 0.5, 5, 10 mg/ml |

| | | |
|---|---|---|
| **HPLC: analyzed by the joint equipment research lab of Dankook University Industry-Academia Cooperation (Citric, pyruvic, succinic, lactic, formic, acetic, propionic, isobutyric, butyric, and isovaleric acid). | | |

### Result 1: Dental plague reduction

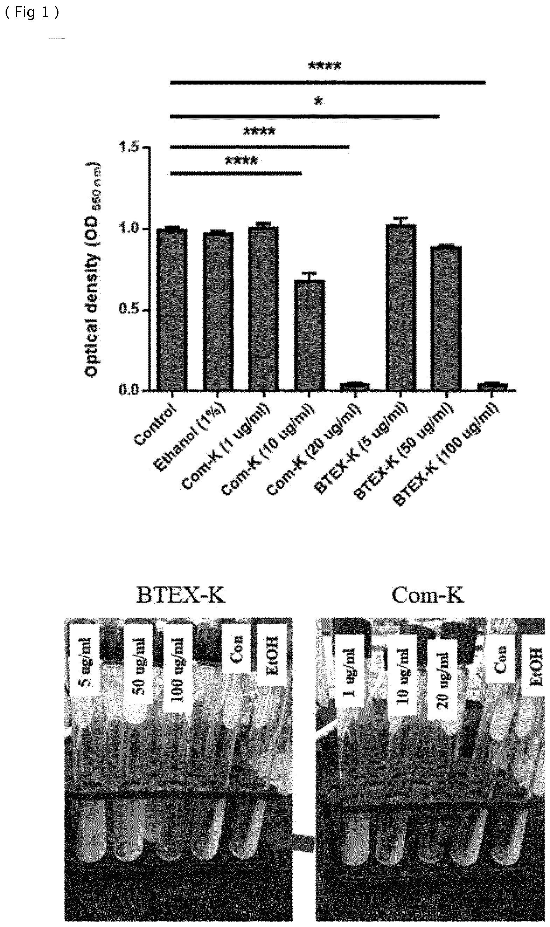

*S. mutans* strain was cultured statically for 24 hours after inoculation while tilted at 30°. When the culture medium was carefully poured out after the static culture, it was observed that bacteria were attached to the wall of the glass tube (FIG. 1, red arrow). Thereafter, the bacteria were suspended by addition of 0.5 M NaOH, and 1 ml of the suspended solution was measured for absorbance at 550 nm. As a result, there was a reduction in dental plaque from a BTEX-K concentration of 50 ug/ml. Particularly, there was a significant reduction at a BTEX-K concentration of 100 µg/ml (p<0.0001).

### Result 2: Amount of organic acid produced in dental plaque

*S. mutans* strain was inoculated into 2 ml of the brain heart infusion (BHI) liquid medium at a concentration of 1 × 10⁵ CFU/ml, and a predetermined concentration of each sample was injected, followed by culturing for 48 hours. The culture was centrifuged at 13,000 rpm for 5 minutes, and then the supernatant was transferred to a new tube and filtered with a 0.45 um syringe filter. Thereafter, HPLC analysis was performed. HPLC analysis conditions are as follows. Mobile phase (Isocratic elution): 10 mM potassium phosphate monobasic (pH 3, a phosphoric acid salt)/MeOH = (98/2), Column oven Temp.: 30°C, flow rate: 1 ml/min, wavelength: 220 nm, and injection amount: 10 µl

As can be seen in the dental plaque reduction experiment, the growth of *S. mutans* strain was inhibited by 100 µg/ml of ginsenoside compound K and 100 ug/ml of BTEX-K.

As a result of HPLC analysis, the peak at 4.6 minutes decreased at these concentrations compared to the control group. When compared to the organic acid standard sample, lactic acid was detected at the same retention time.

A concentration graph of the lactic acid standard sample was created made to analyze the difference in lactic acid content between the control group and experimental groups, followed by statistical processing. As a result, ginsenoside compound K showed an approximately 2-fold reduction in lactic acid content compared to the control group (p<0.0001), and BTEX-K showed an approximately 3.2-fold reduction in lactic acid content compared to the control group (p<0.0001).

Additionally, the peak between 6 and 6.5 minutes in the HPLC result of the control group appeared to decrease for ginsenoside compound K and BTEX-K, but did not match organic acids when compared to organic acid standard samples.

Besides, the peaks detected at the retention time of the 13.5-minute range among the HPLC peaks for ginsenoside compound K and BTEX-K were not detected in the control group.

The dental plaque reduction was observed in ginsenoside compound K and BTEX-K at 20 µg/ml and 100 µg/ml, respectively.

In addition, the inhibition of organic acid production on tooth surfaces was analyzed by HPLC. Ten organic acid standard samples were used, and ginsenoside compound K and BTEX-K were added at different concentrations to liquid media, inoculated with a predetermined amount of *S. mutans,* cultured at 37°C for 48 hours, and then observed for the change in organic acid produced in the culture medium compared to the control. As a result, there were differences in the HPLC results in the liquid cultures with a high concentration (100 ug/ml) of the red ginseng extracts added, ginsenoside compound K and BTEX-K compared to the control group.

The peaks detected at the HPLC retention time of around 4.6 minutes decreased in the cultures with ginsenoside compound K and BTEX-K added. As a comparison result with standard samples, the peaks detected at the retention time of around 4.6 minutes was identified as lactic acid.

### Industrial Applicability

The ginsenoside compound K or complex ginsenoside composition of the present invention can be used as oral compositions including toothpastes and mouthwashes, and can also be applied to food compositions, such as gum, beverages, and snacks.

## Claims

1. A composition for reducing dental plaque and inhibiting dental plaque acidification, the composition comprising, as an active ingredient,
(A) ginsenoside compound K; or
(B) a complex ginsenoside composition containing, relative to 100 parts by weight of the ginsenoside compound K, ginsenoside Rg3 (R+S), ginsenoside F2, ginsenoside Rh2, and ginsenoside Rb1 at least 7 parts or more by weight each.

2. The composition of claim 1, wherein the complex ginsenoside composition B) is obtained by steps:
1) subjecting red ginseng to extraction and concentration to obtain a red ginseng concentrate;
2) dissolving a part of the red ginseng concentrate, followed by adsorption through a synthetic adsorption resin column and then desorption with ethanol, thereby obtaining a complex ginsenoside containing ginsenosides Rb1, Rb2, Rc, and Rd;
3) treating the complex ginsenoside with alpha-galactosidase to obtain a fermented complex ginsenoside; and
4) adding the fermented complex ginsenoside obtained in step 3) to the rest of the red ginseng concentrate obtained in step 1) to obtain a complex ginsenoside composition.

3. The composition of claim 1, wherein the complex ginsenoside composition contains ginsenoside compound K at least 100 mg/g or more, and ginsenoside Rg3 (R+S), ginsenoside F2, ginsenoside Rh2, and ginsenoside Rb1 at least 8 mg/g or more each.

4. The composition of claim 2, wherein the red ginseng concentrates in steps 1), 2), and 4) are in a powder form or a liquid phase.

5. The composition of claim 1 or 2, wherein the ginsenoside compound K or complex ginsenoside composition is in a liquid phase, a cream phase, a paste phase, or a solid phase.

6. A toothpaste composition comprising the composition for reducing dental plaque and inhibiting dental plaque acidification containing a complex ginsenoside composition of any one of claims 1 to 4.

7. A mouthwash composition comprising the composition for reducing dental plaque and inhibiting dental plaque acidification containing a complex ginsenoside composition of any one of claims 1 to 4.

8. A food composition comprising the composition for reducing dental plaque and inhibiting dental plaque acidification containing a complex ginsenoside composition of any one of claims 1 to 4.

9. A pharmaceutical composition comprising the composition for reducing dental plaque and inhibiting dental plaque acidification of any one of claims 1 to 4.
